# EUROPÄISCHE PATENTSCHRIFT

(11) **EP 1 161 408 B1**
(45) Veröffentlichungstag und Bekanntmachung des Hinweises auf die Patenterteilung: **02.01.2004**
(21) Anmeldenummer: 00907651.4
(22) Anmeldetag: 01.03.2000
(51) Int. Cl.: C07C 45/29, C07C 49/39

(54) **VERFAHREN ZUR HERSTELLUNG VON CYCLOBUTANON**
METHOD OF PRODUCING CYCLOBUTANONE
PROCEDE DE PRODUCTION DE CYCLOBUTANONE

(30) Priorität: 10.03.1999 DE 19910464
(43) Veröffentlichungstag der Anmeldung: 12.12.2001
(73) Patentinhaber: Bayer Chemicals AG, 51368 Leverkusen (DE)
(72) Erfinder: KNOPS, Hans-Joachim, D-40789 Monheim (DE); GALLENKAMP, Bernd, D-42113 Wuppertal (DE); MULDER, Lubbertus, D-58135 Hagen (DE); ANTONS, Stefan, D-51373 Leverkusen (DE)
(86) Internationale Anmeldenummer: PCT/EP2000/001707
(87) Internationale Veröffentlichungsnummer: WO 2000/053553

(56) Entgegenhaltungen:
- COLEMAN, K. ET AL: "Selective Catalytic Oxidation of Alcohols by a Ruthenium-Copper Bifunctional System Using Molecular Oxygen" EUR. J. INORG. CHEM., Bd. 11, 1998, Seiten 1673-1675, XP000909457 Weinheim, DE
- ANON.: "A process for making diketone from diol" RESEARCH DISCLOSURE, Nr. 380, 1995, Seiten 816-820, XP000549835 Emsworth, GB
- JORNA ET AL: 'Heterogenization of a ruthenium catalyst on silica and its application in alcohol oxidation and stilbene epoxidation' REACTIVE AND FUNCTIONAL POLYMERS Nr. 29, 1996, Seiten 101 - 114

## Beschreibung

Die Erfindung betrifft ein neues Verfahren zur Herstellung von Cyclobutanon.

Es ist bekannt, dass man Cyclobutanon durch Oxidation von Cyclobutanol erhält. Die beschriebenen Verfahren verwenden dabei übliche organische Oxidationsmittel, wie diverse Dialkyldioxirane, Chloral, tert.-Butylhydroperoxid oder gegebenenfalls substituierte Perbenzoesäuren; bzw. Übergangs- oder Edelmetall-Katalysatoren, einschließlich deren Oxide und Oxidkomplexe (vgl. z.B. React. Funct. Polym., 29 (2), 101-14, 1996; Can. J. Chem., 62 (9), 1835-9, 1984; Org. Synth., 60, 20-5, 1981; and JACS, 96 (21), 6647-57, 1974).

'A process for making Diketone from Diol', Anon, Research Disclosure, Nr. 380, 1955, Seiten 816-820, Emsworth, GB, offenbart die Herstellung von Cyclohexanonderivaten aus Cyclohexanolderivaten mittels Natriumhypochlorit in Gegenwart von Essigsäure.

Für eine Durchführung im großtechnischen Maßstab kommen aus verfahrenstechnischer Sicht allenfalls die Systeme Chrom-VI-Oxid / Oxalsäure und Rutheniumoxid / Natriumperiodat infrage. Aber auch diese haben den generellen Nachteil der zu hohen Kosten des Katalysatorsystems (hier Rutheniumoxid) bzw. der großen Abfallmengen, einschließlich der Entsorgung (hier Chrom und Jod).

Weitere bekannte, nichtoxidative Herstellungsmethoden, wie Photodekomposition, Photooxidation oder Cyclisierungen, gegebenenfalls mit nachfolgender Hydrolyse hochderivatisierter Carbonylfunktionen eignen sich nur für den Labormaßstab.

Es wurde gefunden, dass man Cyclobutanon erhält, wenn man Cyclobutanol mittels Alkali- oder Erdalkalihypochlorit in Gegenwart einer organischen oder anorganischen Säure als Lösungsmittel oxidiert.

Überraschenderweise kann nach dem erfindungsgemäßen Verfahren Cyclobutanon auf einfache Art in sehr guten Ausbeuten und in hoher Reinheit erhalten werden, ohne dass der Cyclobutanring in einer BAYER-VILLIGER-Oxidation zum Butyrolacton als Hauptreaktion erweitert und geöffnet wird.

Die erfindungsgemäße Umsetzung hat somit den Vorteil eines einfachen, kostengünstigen und umweltschonenden Zugangs zu Cyclobutanon, insbesondere im großtechnischen Maßstab.

Verwendet man beispielsweise das System Chlorlauge/Essigsäure zur Oxidation, so kann der Reaktionsablauf des erfindungsgemäßen Verfahrens durch das folgende Formelschema skizziert werden: 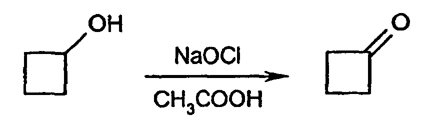 Cyclobutanol als Ausgangsstoff ist eine allgemein bekannte Verbindung der organischen Chemie und in allgemein bekannter Art und Weise erhältlich, wie z.B. durch Umsetzung/Ringerweiterung von Cyclopropylcarbinol mit konzentrierter Salzsäure (vgl. z.B. Org. Synth. 60, 20-25, 1981).

Als Alkali- und Erdalkalihypochlorit seien vorzugsweise Natrium-, Kalium- und Calciumhypochlorit genannt. Die Hypochlorite werden üblicherweise als wäßrige Lösungen eingesetzt. In der Regel eignen sich die technischen Lösungen. Für die technische wäßrige Lösung von Natriumhypochlorit wird auch die Bezeichnung Chlorlauge verwendet.

Als organische Säuren kommen vorzugsweise Alkansäuren, insbesondere C₁-C₄-Alkansäuren, wie Essigsäure infrage; als anorganische Säuren kommen vorzugsweise Mineralsäuren, insbesondere Salzsäure in Frage.

Besonders vorteilhaft ist das System Chlorlauge/Eisessig.

Die Reaktionstemperaturen können bei der Oxidation in einem größeren Bereich variiert werden. Im allgemeinen arbeitet man zwischen -20°C und +30°C, vorzugsweise zwischen -20°C und +20°C, besonders bevorzugt zwischen -10°C und +10°C.

Die Aufarbeitung erfolgt auf übliche Art und Weise, wobei gegebenenfalls durch Zugabe von Alkalicarbonat oder Alkalihydrogencarbonat, wie insbesondere Kaliumoder Natriumhydrogencarbonat der pH-Wert im leicht basischen Bereich gehalten wird (vgl. auch die Herstellungsbeispiele).

In einer besonderen Durchführungsform des erfindungsgemäßen Verfahrens können die Herstellung des Ausgangsstoffes Cyclobutanol aus Cyclopropylcarbinol in Anwesenheit von Säuren, insbesondere konzentrierten Mineralsäuren, und die anschließende Oxidation zum Cyclobutanon in einer Art "Eintopfreaktion" durchgeführt werden, d.h. in situ ohne Isolierung des Zwischenproduktes und ohne Lösemittelwechsel (vgl. auch die Herstellungsbeispiele).

Die Herstellung von Cyclobutanol aus Cyclopropylcarbinol ist an sich bekannt. Sie wird in der Regel in Wasser als Lösemittel in Gegenwart von Säuren, insbesondere Mineralsäuren wie konzentrierte Salzsäuren durchgeführt. Typische Reaktionstemperaturen liegen im Bereich 20°C bis 120°C; in der Regel wird die Reaktion unter Rückflußbedingungen durchgeführt.

Das nach dem erfindungsgemäßen Verfahren herzustellende Cyclobutanon stellt ein allgemein interessantes, zentrales Zwischenprodukt dar.

### Herstellungsbeispiele

### Beispiel 1

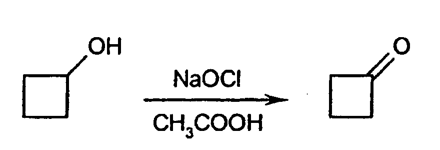

6,3 kg (76,1 Mol; 84 % Gehalt) Cyclobutanol werden in 23,6 1 Essigsäure unter Rühren gelöst und innerhalb von 5 Stunden bei 0°C bis 5°C (Manteltemperatur - 18°C) 42 1 Chlorlauge (13 % aktives Chlor) zudosiert. Die gelbliche Suspension wird 12 Stunden (über Nacht) bei 3°C bis 10°C gerührt und anschließend in 87 1 Wasser eingerührt, wobei sich der pH-Wert 3 einstellt.

Mit je 2 x 30 l und 2 x 16 l Methylenchlorid wird extrahiert, wobei die wässrige Phase klar wird. Die organische Phase wird in 60 1 Wasser eingerührt und langsam (Schäumen!) unter Rühren 6,9 kg Natriumhydrogencarbonat zudosiert, wobei sich der pH-Wert 6 einstellt. Die wässrige Phase wird abgetrennt, die organische Phase mit 30 1 Wasser versetzt und erneut unter Rühren 2,3 kg Natriumhydrogencarbonat zugegeben, wobei der pH-Wert 8 erreicht wird.

Die organische Phase wird abgetrennt und im Kessel bei 40°C und 550 bis 600 mbar eingeengt. Der Rückstand (18,2 kg) wird im Destillationslabor feindestilliert.

Man erhält 3,52 kg (64,75 % der Theorie) Cyclobutanon vom Siedepunkt 88°C / 750 mbar und einer Reinheit von 98 % / GC.

### Beispiel 2

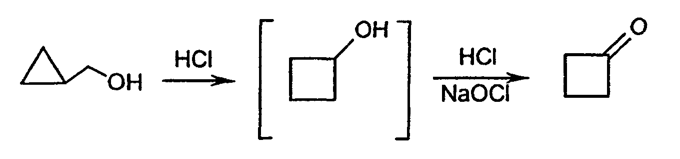

73,1 g (1 Mol) Cyclopropylcarbinol in 680 ml Wasser werden mit 100 ml konzentrierter Salzsäure versetzt und 3 Stunden unter Rückfluß erhitzt. Das Reaktionsgemisch wird anschließend bei 0°C bis 5°C innerhalb von 3 Stunden tropfenweise mit 819 g Chlorlauge (13 % aktives Chlor) versetzt, wobei der pH-Wert nicht über 2 steigen soll (gegebenenfalls Salzsäure-Zugabe). Man läßt bei 0°C bis 5°C ca. 4 Stunden nachrühren und extrahiert zweimal mit je 250 ml Dichlormethan. Die vereinigten Extrakte werden durch Abdestillieren des Lösungsmittels eingeengt und der Rückstand unter Normaldruck destilliert.

Man erhält 59,4 g (84,7 % der Theorie) Cyclobutanon mit einer Reinheit von 95,5 % / GC.

## Patentansprüche

1. Verfahren zur Herstellung von Cyclobutanon, bei dem man Cyclobutanol mittels Alkali- oder Erdalkalihypochlorit in Gegenwart einer Säure oxidiert.

2. Verfahren gemäß Anspruch 1, **dadurch gekennzeichnet, dass** man als Säuren eine C₁-C₄-Alkansäure einsetzt.

3. Verfahren gemäß Anspruch 1, **dadurch gekennzeichnet, dass** man Natriumhypochlorit einsetzt.

4. Verfahren gemäß Anspruch 1, **dadurch gekennzeichnet, dass** man bei einer Temperatur von -20°C bis +30°C arbeitet.

5. Verfahren gemäß Anspruch 1, bei dem man zunächst Cyclobutanol durch Umlagerung von Cyclopropylcarbinol in Gegenwart von Säuren herstellt und dann in situ zu Cyclobutanon oxidiert.

## Claims

1. Process for preparing cyclobutanone in which cyclobutanol is oxidized by means of alkali metal hypochlorite or alkaline earth metal hypochlorite in the presence of an acid.

2. Process according to Claim 1, **characterized in that** a C₁-C₄-alkanoic acid is used as acid.

3. Process according to Claim 1, **characterized in that** sodium hypochlorite is used.

4. Process according to Claim 1, **characterized in that** the oxidation is carried out at a temperature of from -20°C to +30°C.

5. Process according to Claim 1, in which cyclobutanol is firstly prepared by rearrangement of cyclopropyl carbinol in the presence of acids and is then oxidized in situ to form cyclobutanone.

## Revendications

1. Procédé pour la préparation de la cyclobutanone selon lequel on oxyde le cyclobutanol à l'aide d'un hypochlorite alcalin ou alcalino-terreux en présence d'un acide.

2. Procédé selon la revendication 1, **caractérisé en ce que** l'acide utilisé est un acide alcanoïque en C₁ à C₄.

3. Procédé selon la revendication 1, **caractérisé en ce que** l'on utilise l'hypochlorite de sodium.

4. Procédé selon la revendication 1, **caractérisé en ce que** l'on opère à une température comprise entre -20 et +30°C.

5. Procédé selon la revendication 1, **caractérisé en ce que** l'on prépare d'abord le cyclobutanol par transposition du cyclopropylcarbinol en présence d'un acide puis on oxyde in situ en la cyclobutanone.
